# DEMANDE DE BREVET EUROPEEN

(11) **EP 2 384 698 A1**
(43) Date de publication de la demande: **09.11.2011**
(21) Numéro de dépôt: 11172205.4
(22) Date de dépôt: 12.06.2008
(51) Int. Cl.: A61B 5/15, G01T 1/161, A61B 6/00

(54) **Méthode de comptage de particules élémentaires émises par un fluide dans un conduit**

(30) Priorité: 19.06.2007 FR 0704348
(62) Demande divisionnaire de: 08290543.1
(71) Demandeur: COMMISSARIAT A L'ENERGIE ATOMIQUE ET AUX ENERGIES ALTERNATIVES, 75015 Paris (FR)
(72) Inventeur: Reymond, Jean-Marc, 78470 SAINT-REMY-LES-CHEVREUSE (FR); Kerhoas-Cavata, Sophie, 78125 RAIZEUX (FR); Mangeot, Philippe, 94270 LE KREMLIN-BICÊTRE (FR)
(74) Mandataire: Bolinches, Michel Jean-Marie

(57) **Abrégé**

L'invention concerne une méthode de comptage de particules élémentaires émises par un fluide qui est mise en oeuvre par un dispositif de comptage comportant un conduit de transfert du fluide et, à l'extérieur du conduit, des moyens de détection (6a) des particules qui sont atténuées par une paroi du conduit et/ou par ce fluide.

Ce dispositif comprend au moins un tronçon de comptage (4a) de section transversale oblongue qui relie entre elles deux portions adjacentes du conduit présentant une plus grande section de passage et qui présente un ratio [hauteur interne (h) / largeur interne (I)] inférieur ou égal à 20 %, où la hauteur et la largeur internes représentent respectivement la plus petite et la plus grande des dimensions transversales du tronçon mesurées selon deux directions sensiblement perpendiculaires, ces moyens de détection s'étendant transversalement à ce tronçon en regard de toute sa largeur interne et de part et d'autre de celle-ci.

Cette méthode est telle que l'on choisit pour la paroi dudit tronçon de comptage un matériau qui est sensiblement transparent à la longueur d'onde des particules émises par ledit fluide.

## Description

La présente invention concerne un dispositif et une méthode de comptage de particules élémentaires (telles que des particules α, γ, des électrons ou des positons) émises par un fluide, le dispositif comportant un conduit de transfert de ce fluide et, à l'extérieur du conduit, des moyens de détection de ces particules qui sont atténuées par une paroi du conduit et/ou par ce fluide. L'invention s'applique plus particulièrement, mais non exclusivement, à un fluide constitué de microéchantillons sanguins d'un mammifère.

De manière connue, le comptage de particules atténuées par certains composants d'un fluide les incorporant et/ou de la paroi du conduit transférant ce fluide pose certains problèmes techniques, qui sont particulièrement pénalisants dans le cas particulier du comptage au sein de petits échantillons de fluides, dans le cas d'absorption de ces particules par le fluide, ou bien lorsque les particules à compter sont des électrons ou des positons émis par radioactivité bêta, notamment. Parmi les applications cumulant ces trois problèmes, on peut principalement citer celles qui mesurent directement les positons ou les électrons issus de la radioactivité bêta contenue dans des microéchantillons sanguins, par exemple pour les dispositifs de mesure de la fonction d'entrée d'un mammifère destinés à l'imagerie quantitative.

Des laboratoires comme celui de Sherbrooke au Canada ont développé des détecteurs de positons ou d'électrons pour des microéchantillons sanguins incorporant des émetteurs bêta à titre de radiotraceurs, en proposant des dispositifs de comptage dont l'efficacité des détecteurs ne dépasse pas 7 % dans le cas de radiotraceurs au ¹⁸F et 16 % dans le cas de radiotraceurs au ¹¹C. Ces dispositifs comprennent un conduit tubulaire de section de passage constante sur toute sa longueur, sans particularité aucune dans sa zone de détection pourvue des détecteurs. Ce conduit présente un diamètre interne de 0,58 mm, et comme le libre parcours moyens des positons dans le sang est de l'ordre de 0,6 mm pour le ¹⁸F, il en résulte que peu de positons sortent de ces microéchantillons sanguins. De plus, le matériau utilisé pour ce conduit - un polyéthylène de basse densité (PEBD) - présentant une épaisseur de paroi e très élevée (e ≥ 150 µm et typiquement égale à 192,5 µm) pour une masse volumique d proche de 1 g/cm³ (typiquement égale à 0,92 g/cm³ pour du « PE10 »), il en résulte que ce conduit génère une atténuation des électrons ou des positons qui est relativement élevée, étant proportionnelle de manière connue au produit e.d qui est ici typiquement égal à 192,5 x 0,92 soit à environ 177.

Sont également connus sous la dénomination « β-microprobe » d'autres méthodes et dispositifs de comptage de particules qui sont utilisés par la société française Biospace pour la mesure de la fonction d'entrée d'un petit mammifère, tel qu'un rat ou une souris. Ces méthodes consistent essentiellement à éliminer le conduit précité en introduisant directement le détecteur dans une artère de l'animal, à l'état immergé dans le sang de ce dernier.

Un inconvénient majeur de cette solution sans conduit est que le radiotraceur accumulé dans les autres organes de l'animal crée une forte perturbation par rayonnements gamma. Un autre inconvénient de cette solution réside dans les faibles dimensions caractérisant la mesure, ce qui conduit à une faible efficacité de comptage. Il est alors nécessaire d'adjoindre au détecteur principal un second détecteur, que l'on place dans le corps de l'animal mais hors du sang de celui-ci et que l'on utilise de manière différentielle pour soustraire le bruit de fond dû à ces rayonnements gamma.

Même dans ces conditions de mesure, il s'avère que l'efficacité de détection (réduite à l'absorption dans le sang) annoncée est seulement de 16 % et de 20 % pour des radiotraceurs à base de ¹⁸F et de 11C, respectivement, avec un détecteur plastique de type scintillant de 1 mm de diamètre.

Etant donné l'évolution récente des technologies vers le traitement de microéchantillons et la volonté des biologistes de développer des outils destinés aux petits animaux, tels que des souris, il est fondamental de pouvoir mesurer l'activité de microéchantillons liquides contenant le moins possible de radiotraceurs. Ces microéchantillons présentant un volume de l'ordre de quelques µL (typiquement de 8 à 30 µL), il est nécessaire que les dispositifs de comptage utilisés présentent une efficacité (i.e. une sensibilité de mesure) élevée, particulièrement pour l'étude de radiotraceurs à vie courte comme le ¹¹C, dont l'activité en fin de séquence diminue de façon significative (la demi-vie du ¹¹C étant de 20 minutes).

Un but de la présente invention est de proposer un dispositif de comptage de particules élémentaires émises par un fluide, le dispositif comportant un conduit de transfert de ce fluide et, agencé à l'extérieur du conduit, des moyens de détection de ces particules, lesquelles sont atténuées par une paroi du conduit et/ou par ce fluide, dispositif qui remédie aux inconvénients précités en présentant une sensibilité de mesure améliorée.

A cet effet, un dispositif de comptage selon l'invention est tel qu'il comprend au moins un tronçon de comptage de section transversale oblongue qui relie entre elles deux portions adjacentes de ce conduit présentant une plus grande section de passage et qui présente un ratio [hauteur interne / largeur interne] inférieur ou égal à 20 %, où la hauteur et la largeur internes représentent respectivement la plus petite et la plus grande des dimensions transversales du tronçon mesurées selon deux directions sensiblement perpendiculaires, lesdits moyens de détection s'étendant transversalement à ce tronçon en regard de toute sa largeur interne et de part et d'autre de celle-ci.

On notera que dans le cas où les particules à compter sont des électrons ou des positons, cette géométrie extrêmement aplatie du tronçon de comptage combinée à sa largeur interne relativement élevée en regard de laquelle sont agencés en dépassement lesdits moyens de détection procure une efficacité de comptage très élevée, typiquement supérieure à 50 % tant pour des radiotraceurs à base de ¹⁸F que de ¹¹C, permettant ainsi des mesures même sur des microéchantillons de sang de volume inférieur à 10 µL (typiquement prélevés sur des souris) et pouvant contenir des radiotraceurs à vie courte (tels que le ¹¹C).

On notera en particulier que cet agencement desdits moyens de détection à la fois en regard et en dépassement de la largeur transversale du tronçon de comptage permet de maximiser l'acceptance géométrique de ces moyens de détection (i.e. de maximiser l'ensemble des directions de l'espace selon lesquelles une molécule de radiotraceur peut émettre une particule élémentaire dont le trajet impacte les moyens de détection, rendant possible sa « capture »), contribuant ainsi à améliorer cette efficacité de comptage.

Par « section de passage », on entend dans la présente description la section transversale interne du tronçon de comptage (de forme oblongue ou aplatie) et celle de chacune des deux portions adjacentes à ce tronçon (de préférence circulaire).

Lorsque le fluide est un liquide, ce tronçon de comptage présente préférentiellement un ratio [hauteur interne / largeur interne] compris entre 5 % et 10 %.

Avantageusement, le ratio de la section de passage du tronçon sur celle de chaque portion adjacente peut être inférieur ou égal à 35 % et, encore plus avantageusement, être inférieur à 25 %.

Egalement avantageusement, ladite hauteur interne du tronçon de comptage peut être inférieure à 20 % du diamètre interne de chacune des portions cylindriques adjacentes, et ladite largeur interne de ce tronçon peut être supérieure à 1,3 fois ce diamètre interne.

Selon un mode préférentiel de réalisation de l'invention, ce tronçon de comptage présente une section transversale sensiblement rectangulaire dont les grands côtés et/ou les petits côtés sont incurvés selon des courbures symétriques l'une de l'autre, de sorte que ce tronçon présente au moins en partie une face externe sensiblement convexe ou concave.

Selon une autre caractéristique préférentielle de l'invention, ledit conduit est adapté à la circulation de microéchantillons liquides, tels que des microéchantillons sanguins, ladite hauteur interne du tronçon de comptage étant comprise entre 100 µm et 250 µm et ladite largeur interne de ce tronçon étant supérieure à 1,3 mm, alors que la ou chaque portion cylindrique adjacente présente un diamètre compris entre 0,8 mm et 1,2 mm.

Par « microéchantillons », on entendra dans la présente description des échantillons liquides présentant chacun un volume inférieur à 100 µL et, de préférence, inférieur ou égal à 30 µL (i.e. typiquement des échantillons prélevés sur des petits animaux).

Encore plus préférentiellement, l'aire de la section de passage dudit tronçon de comptage est comprise entre 0,15 mm² et 0,25 mm², et ce tronçon peut alors présenter une longueur comprise entre 30 mm et 40 mm de sorte à pouvoir contenir un microéchantillon d'environ 8 µL en regard desdits moyens de détection.

Spécifiquement lorsque les particules à compter sont des électrons ou des positons issus de la radioactivité bêta émise par ledit fluide, ledit tronçon de comptage présente avantageusement une paroi d'épaisseur e, exprimée en µm, et de masse volumique d, exprimée en g/cm³, dont le produit e.d est inférieur à 100 et de préférence inférieur à 50, de telle sorte que l'atténuation par ce tronçon des particules à compter soit minimisée.

Selon une autre caractéristique avantageuse de l'invention qui concerne notamment de telles particules de type électrons ou positions, ledit tronçon de comptage est à base d'un polymère thermoformé de masse volumique inférieure ou égale à 1,5 g/cm³, de préférence un polyimide de dénomination « Kapton », et ce tronçon présente une paroi d'épaisseur inférieure à 50 µm et de préférence inférieure à 30 µm. De préférence, ce matériau est choisi biocompatible si le liquide mesuré est biologique et susceptible d'être réinjecté à un être vivant.

On notera que cette sélection d'un tel polyimide de type « Kapton » permet d'obtenir cette valeur très faible pour le produit e.d précité, en comparaison des valeurs usuelles de ce produit généralement comprises entre 150 et 200 pour les conduits connus réalisés en PEBD, qui présentent une densité inférieure à celle de ce polyimide « Kapton » mais une épaisseur très nettement supérieure.

Selon une autre caractéristique de l'invention, lesdits moyens de détection comportent avantageusement deux ensembles de détecteurs respectivement disposés contre ou à proximité immédiate de deux grandes faces sensiblement planes dudit tronçon de comptage qui sont distantes entre elles de ladite hauteur et qui sont reliées entre elles par deux petites faces de ce tronçon, lesquelles sont dépassées dans le sens de ladite largeur par ces ensembles de détecteurs afin de maximiser l'acceptance géométrique de ces derniers.

Selon un mode particulier de réalisation de l'invention, ledit dispositif peut être agencé en amont d'une pompe péristaltique pilotée par ordinateur pour l'aspiration par à-coups d'une quantité déterminée d'échantillons à prélever, lesdites grandes faces dudit tronçon de comptage présentant alors chacune en leur côté externe une forme qui est avantageusement de type légèrement convexe.

On notera que cette géométrie convexe des grandes faces externes du tronçon de comptage est avantageusement utilisable de manière générale lorsque le fluide circulant dans le conduit est soumis à une surpression ou à une pression qui varie dans le temps, par exemple en cas d'aspirations successives des échantillons prélevés.

La méthode de comptage de particules élémentaires selon l'invention est mise en oeuvre au moyen d'un dispositif de comptage tel que défini ci-dessus, et cette méthode consiste notamment à choisir pour la paroi dudit tronçon de comptage un matériau qui est sensiblement transparent aux particules émises par ledit fluide.

Selon un premier exemple de réalisation de l'invention, ces particules sont des photons émis par fluorescence, et dans ce cas la paroi de ce tronçon doit être choisie transparente à la longueur d'onde des photons considérés.

Selon un second exemple de réalisation de l'invention, ces particules sont des électrons ou des positons émis par radioactivité bêta, ladite paroi présentant une épaisseur e, exprimée en µm, et une masse volumique d, exprimée en g/cm³, telle que le produit e.d soit inférieur à 100 et de préférence inférieur à 50, de sorte à minimiser l'absorption de ces particules afin de ne pas affecter significativement le comptage.

Avantageusement, cette méthode de comptage selon l'invention est telle que ledit fluide est constitué de microéchantillons sanguins qui circulent dans ledit conduit et dans le plasma desquels est dilué un radiotraceur bêta à vie courte, tel que le carbone 11.

Encore plus avantageusement selon cette méthode de l'invention, ces microéchantillons ont été successivement prélevés dans un mammifère, tel qu'un rat ou une souris, et présentent chacun un volume inférieur à 100 µL et de préférence inférieur ou égal à 30 µL, la totalité du volume de chaque microéchantillon étant sensiblement localisée dans ledit tronçon de comptage en regard desdits moyens de détection, pour que ces microéchantillons se succèdent dans ce tronçon de manière discrétisée dans l'espace et dans le temps.

On notera que la petite taille des animaux qui sont de préférence utilisés pour la mesure des microéchantillons prélevés requiert de limiter le volume total de ces microéchantillons à une quantité qui est compatible non seulement avec la santé de l'animal, mais encore avec une perturbation aussi faible que possible de son métabolisme.

Les caractéristiques précitées de la présente invention, ainsi que d'autres, seront mieux comprises à la lecture de la description suivante de plusieurs exemples de réalisation de l'invention, donnés à titre illustratif et non limitatif, ladite description étant réalisée en relation avec les dessins joints, parmi lesquels :
la figure 1 est une vue schématique partielle d'un système automatisé de prélèvement et de mesure selon l'invention incluant un dispositif de comptage de radioactivité par exemple bêta, agencé en amont d'un dispositif d'échantillonnage des microéchantillons prélevés,
la figure 2 est une photographie illustrant la forme et les dimensions relatives, en comparaison d'une pièce de 10 centimes d'euro, d'un conduit de ce système de prélèvement et de mesure incluant un tronçon aplati apte à optimiser ce comptage de radioactivité,
la figure 3 est une vue schématique en section transversale de ce tronçon aplati selon l'invention équipé des deux ensembles de détecteurs illustrés à la figure 1, et
la figure 4 est un graphique illustrant l'efficacité de détection en radiotraceur ¹⁸F en fonction du seuil de détection, pour trois types de conduits de prélèvement comprenant ce tronçon de comptage aplati selon l'invention et, à titre d'essais comparatifs, deux micro-tubes cylindriques.

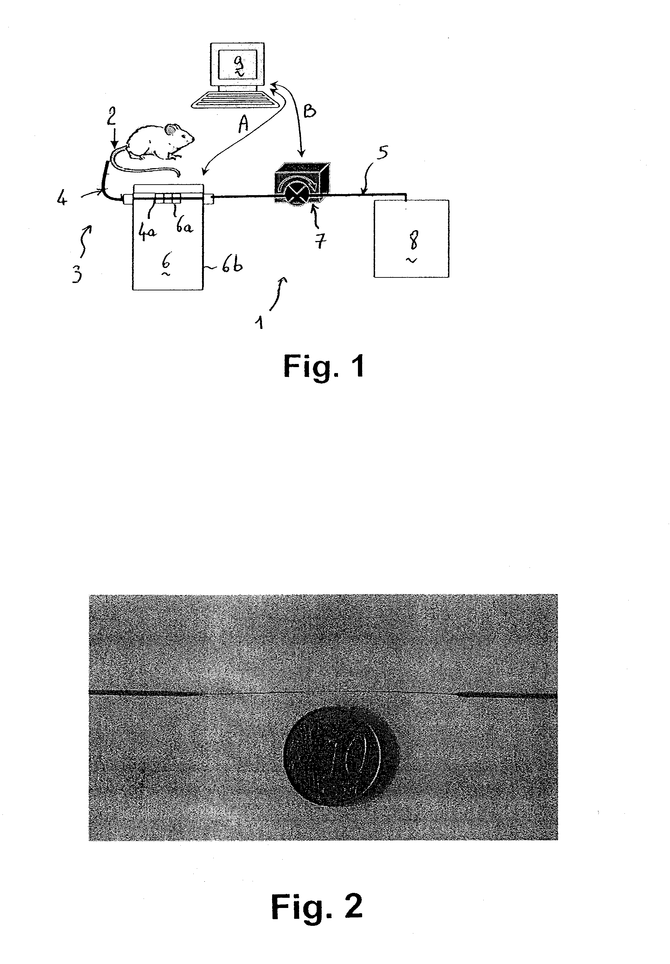

La figure 1 illustre à titre d'exemple une installation 1 automatisée pour procéder successivement et en continu au prélèvement, au stockage temporaire et à des mesures radiatives de microéchantillons sanguins d'un petit mammifère 2 par exemple de type rat ou souris, au moyen d'un système de prélèvement 3 incluant une succession de conduits 4 et 5 de type micro-tubes ou capillaires souples. Ce système de prélèvement 3 comporte essentiellement :
- un cathéter équipé d'un dispositif de raccordement (non illustrés) et qui est destiné à aspirer par à-coups une même quantité de sang à prélever, via une pompe péristaltique 7,
- un dispositif de comptage 6 de particules présentes dans les microéchantillons prélevés, qui est dans cet exemple un compteur de positons ou d'électrons issus de la radioactivité bêta 6 pour des microéchantillons de sang total et qui est placé au plus près du point de prélèvement en étant quasiment au contact d'un tronçon de comptage 4a de cette succession de conduits 4, 5 (comme développé ci-après, ce tronçon 4a présente des caractéristiques de forme et de matériau optimisées pour ce comptage et est centré par rapport aux diodes de détection 6a que comporte le dispositif 6),
- un système d'échantillonnage 8 agencé en aval de la pompe péristaltique 7, où ces microéchantillons prélevés et analysés sont stockés et traités, et
- un dispositif de commande 9 assisté par ordinateur pour la commande de l'ensemble du système 3, en incluant cette pompe 7 (voir les doubles flèches A et B à la figure 1 pour cette commande).

Selon l'invention, la succession de conduits 4 et 5 est avantageusement telle que les élargissements de section transversale présents sur ces conduits sont tous inférieurs ou égaux à 20 % en termes de ratios de surfaces, de sorte que les microéchantillons se suivant dans cette succession de conduits 4 et 5 ne s'y mélangent pratiquement pas entre eux par diffusion. De cette manière, ces microéchantillons prélevés sont discrétisés dans l'espace et dans le temps.

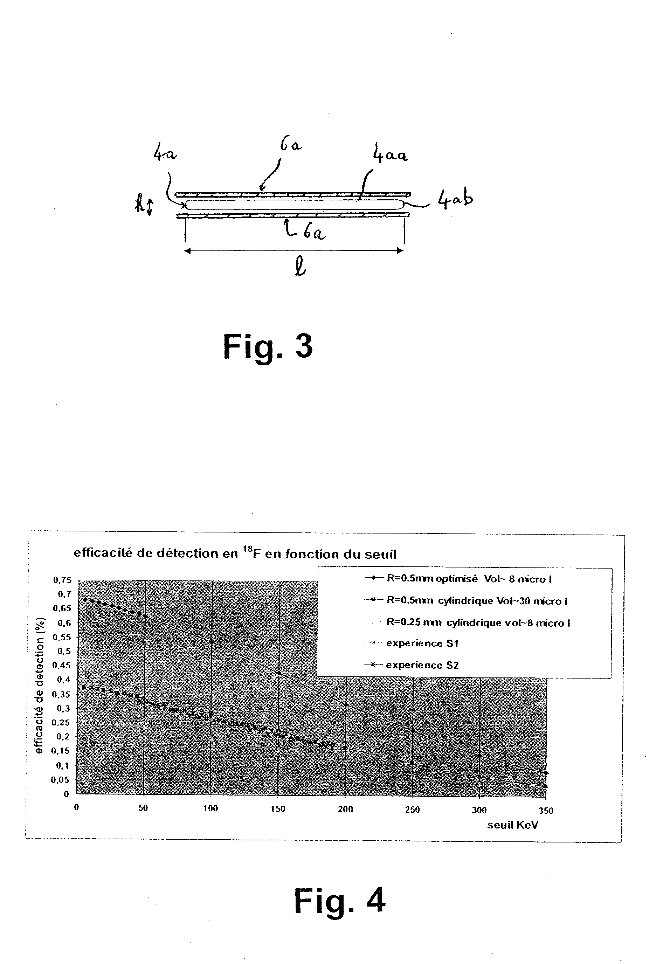

Comme illustré aux figures 2 et 3, on a prévu dans la succession de conduits de prélèvement 4, 5 selon l'invention un tronçon de comptage aplati 4a qui relie entre elles deux portions cylindriques et qui est conçu pour optimiser le comptage de particules par le dispositif 6 de la figure 1 (tel qu'un compteur bêta, que l'on utilise avantageusement pour la mesure de la fonction d'entrée du petit mammifère 2). On a ainsi choisi de réduire le volume de détection et d'augmenter l'efficacité de comptage.

A cet effet, on a façonné par thermoformage ce tronçon aplati 4a de section transversale oblongue qui est réalisé de préférence en un polyimide de dénomination « Kapton » (de masse volumique égale à 1,42 g/cm³, avec une paroi de 25 µm ±10 % d'épaisseur) et qui relie entre eux deux micro-tubes cylindriques par exemple en PEBD (polyéthylène de basse densité) de diamètre interne égal à 1 mm environ. Comme illustré à la figure 3, les diodes de détection 6a du dispositif 6 de comptage sont agencées de part et d'autre des faces externes des grands côtés 4aa du tronçon 4a, par rapport à sa plus petite dimension transversale constituée ici par sa hauteur h.

Dans cet exemple de réalisation, le tronçon aplati 4a présente une section transversale sensiblement rectangulaire dont les faces externes des petits côtés 4ab sont incurvées selon des courbures convexes symétriques l'une de l'autre, et ce tronçon présente un ratio [hauteur interne h / largeur interne I] d'environ 8 %, où la hauteur et la largeur internes sont respectivement égales à 130 µm et à 1490 µm.

Quant au ratio surfacique de la section de passage du tronçon aplati 4a - d'environ 0,1937 mm² - sur celle de chaque portion cylindrique adjacente (de section interne d'environ 0,785 mm²), il est légèrement inférieur à 25%.

En outre, le tronçon aplati 4a présente une paroi d'épaisseur e et de masse volumique d dont le produit e.d est sensiblement égal à 35,5 (avec e = 25 µm et d = 1,42 g/cm³), ce qui est très nettement inférieur aux valeurs usuellement utilisées qui sont généralement comprises entre 150 et 200 pour les micro-conduits réalisés en PEBD (qui présentent une densité inférieure à celle du « Kapton » mais une épaisseur nettement supérieure), de telle sorte que l'atténuation par ce tronçon 4a selon l'invention des particules à compter, telles que des électrons ou des positons issus de la radioactivité bêta, est nettement minimisée.

Comme illustré à la figure 3, le tronçon aplati 4a est équipé, en regard de ses grandes faces 4aa - qui sont de préférence légèrement convexes - et venant en dépassement de ses petites faces 4ab, de deux ensembles desdites diodes de détection 6a aptes à compter lesdites particules de chaque microéchantillon liquide y circulant (ce dépassement des diodes 6a permet d'optimiser leur acceptance géométrique, et donc la « capture » des particules à compter).

Le procédé de thermoformage utilisé pour l'obtention de ce tronçon aplati 4a selon l'invention comprend notamment les étapes suivantes :
- mise en place du tronçon 4a à froid dans le gabarit de formage,
- raccordement de ses deux extrémités à des micro-tubes souples pour la mise en pression,
- mise sous pression (1,5 bars de pression relative),
- chauffage du gabarit à 300° C pendant 15 minutes,
- refroidissement sous pression, et
- chute lente de la pression après refroidissement.

On obtient par ce thermoformage un tronçon aplati de comptage 4a dont les deux grandes faces 4aa présentent chacune une superficie d'environ 1,5 mm x 35 mm soit 52,5 mm², ce qui se traduit par une minimisation de l'épaisseur de liquide que le positon ou l'électron doit traverser pour atteindre les détecteurs 6a.

Le graphique de la figure 4 illustre, sous forme de courbes de simulation confirmées par l'expérience, les résultats d'efficacité de comptage obtenus pour deux séries d'expériences S1 et S2, réalisées chacune :
- avec un conduit selon l'invention de rayon égal à 0,5 mm pour les portions cylindriques et incorporant ce tronçon 4a aplati, avec un volume de microéchantillon sanguin prélevé de 8 µL (compatible avec une souris),
- avec un premier conduit « témoin » cylindrique sur toute sa longueur (i.e. sans tronçon aplati) présentant un rayon de 0,5 mm, avec un volume de microéchantillon sanguin prélevé de 30 µL (compatible avec un rat), et
- avec un second conduit « témoin » cylindrique sur toute sa longueur (i.e. sans tronçon aplati) présentant un rayon de 0,25 mm, avec un volume de microéchantillon sanguin prélevé de 8 µL.

Grâce à ce tronçon aplati 4a, on note que l'efficacité de détection des positons augmente, passant de 32 % avec les micro-tubes cylindriques à plus de 60 % avec le micro-tube de l'invention, pour le seuil minimum (d'environ 46 Kev). Le gain est même plus important car un conduit micro-tubulaire cylindrique compatible avec 8 µL donnerait environ 25 % d'efficacité. Le micro-tube optimisé selon l'invention permet ainsi de travailler avec des échantillons de 8 µL, en atteignant plus de 60 % d'efficacité au seuil minimum, contre seulement 25 % avec un micro-tube entièrement cylindrique sur sa longueur, ce qui rend le système de prélèvement 3 selon l'invention particulièrement bien adapté à la mesure de la fonction d'entrée d'une souris.

En relation avec la figure 1, on va à présent décrire plus précisément le dispositif et la méthode de comptage des particules bêta utilisés en relation avec le système de prélèvement automatisé 3.

Quelques centimètres en aval du premier conduit 4 de prélèvement, chaque microéchantillon passe au plus près du compteur de particules bêta 6, pour lequel l'épaisseur de la paroi du conduit n'apporte qu'une très faible atténuation. Le tronçon aplati 4a, fixé dans le boîtier 6b du compteur 6, permet de minimiser le taux d'annihilation des positons dans les parois, et sa géométrie est telle qu'il peut contenir le volume d'un échantillon (de 30 µL ou de 8 µL) correctement centré sous les six diodes de détection en silicium (10 x 10 x 0,3 mm³) entourant le tronçon 4a, comme illustré à la figure 1. Ces diodes 6a sont elles-mêmes entourées d'un blindage de plomb de 2 cm d'épaisseur destiné à supprimer le bruit physique venant des photons issus de l'animal 2. Le reste de ce système de mesure 6 comporte une carte électronique de traitement et interfaçage, l'ensemble privilégiant la compacité et la robustesse en formant un boîtier 6b de petites dimensions (8 x 10 x 4 cm³).

Il est avantageux, pour minimiser la probabilité d'annihilation dans le sang d'un positon issu de la radioactivité bêta, de donner au micro-tube souple la forme aplatie du tronçon 4a, au moins à l'endroit de son passage devant les diodes 6a. Par ailleurs, comme expliqué précédemment, cette configuration géométrique assure un étalement du liquide en une nappe fine, ce qui augmente la surface de liquide en regard des surfaces détectrices. La configuration retenue pour le système de mesure 6 est la suivante.

Le tronçon aplati 4a, de 25 µm d'épaisseur de paroi, est placé en « sandwich » entre les diodes 6a de 0,3 mm d'épaisseur (trois diodes 6a en haut et trois autres en bas).

L'électronique de lecture de ces diodes 6a ainsi que l'électronique gérant l'acquisition et le transfert de données ont été intégrées dans un unique module électronique, qui a été optimisé afin de réduire au maximum le bruit électronique permettant de minimiser le seuil de détection pour une meilleure efficacité.

L'électronique « front-end » (mise en forme et discriminateur) est assurée par un «ASIC» (comprenant 16 voies, un seuil commun, 16 sorties + 1 OR). Le seuil est réglable par l'utilisateur. La carte d'acquisition est une carte de test « USB » configurable qui tire parti de la souplesse de l'interface « USB» des ordinateurs personnels et des progrès des circuits numériques configurables « FPGA » (« Field Programmable Gate Array »). Elle permet de traiter rapidement un grand nombre de signaux et est programmable depuis l'interface d'un ordinateur.

Le schéma de principe est illustré à la figure 1. Le conduit de prélèvement 4 se prolonge par le tronçon aplati 4a qui prend le relais à l'intérieur du boîtier 6b, et le sang ressort de l'autre côté de celui-ci. Cette circulation du sang est réalisée grâce à la pompe péristaltique 7. Le volume des microéchantillons prélevés est réglable, ainsi que leur temps de prélèvement. Ces paramètres sont pilotés par l'ordinateur du dispositif de commande 9 de l'installation 1.

L'espacement minimum entre deux prélèvements de microéchantillons est de 1 seconde. Afin de couvrir la dynamique d'une cinétique de radiotraceur dans le sang, les microéchantillons sont prélevés toutes les secondes après l'injection pendant environ 30 secondes à 1 minute, puis ils sont prélevés de façon plus espacée dans le temps, la pente de la courbe étant plus faible pendant cette phase.

Les connexions entre les divers conduits et le boîtier 6b sont conçues pour éviter toute perte de volume des microéchantillons et toute diffusion entre deux microéchantillons adjacents n'est possible.

Ces microéchantillons sanguins sont ainsi prélevés de façon discrète dans l'espace et dans le temps, et ils progressent sans diffuser jusqu'au système d'échantillonnage 8 où ils sont stockés et avantageusement soumis à l'extraction d'une au moins de leurs phases ou de leurs composants, par exemple mise en oeuvre par centrifugation.

On notera que ce système de prélèvement 3 selon l'invention peut être avantageusement utilisé dans le domaine de la recherche préclinique pour l'imagerie quantitative de nouveaux traceurs, notamment en tomographie par émission de positons (TEP). Dans ce cas, le liquide considéré est du sang, et l'application consiste à mesurer la fonction d'entrée pour de petits animaux comme les rats ou les souris. La faible taille de ces animaux, et donc la faible quantité totale de sang qu'ils possèdent, limitent le volume de chaque microéchantillon à environ 30 µL pour les rats, et à environ 8 µL pour les souris.

Une autre application possible de l'invention concerne un fluide gazeux constitué d'air marqué par exemple par un radio isotope du xénon. Dans ce cas le dispositif comprend un tronçon de comptage 4a de section transversale oblongue qui relie entre elles deux portions adjacentes de ce conduit présentant une plus grande section de passage et qui présente un ratio [hauteur interne (h) / largeur interne (I)] égal à 20 %, où la hauteur et la largeur internes représentent respectivement la plus petite et la plus grande des dimensions transversales de ce tronçon mesurées selon deux directions sensiblement perpendiculaires, lesdits moyens de détection s'étendant transversalement à ce tronçon en regard de toute sa largeur interne et de part et d'autre de celle-ci.

## Revendications

1. Méthode non chirurgicale de comptage avec une sensibilité améliorée de particules élémentaires émises par un fluide, **caractérisée en ce qu'**elle est mise en oeuvre au moyen d'un dispositif de comptage comportant un conduit de transfert de ce fluide et, agencé à l'extérieur dudit conduit, des moyens de détection (6a) de ces particules, lesquelles sont atténuées par une paroi dudit conduit et/ou par ce fluide, ce dispositif comprenant au moins un tronçon de comptage (4a) de section transversale oblongue qui relie entre elles deux portions adjacentes de ce conduit présentant une plus grande section de passage et qui présente un ratio [hauteur interne (h) / largeur interne (I)] inférieur ou égal à 20 %, où la hauteur et la largeur internes représentent respectivement la plus petite et la plus grande des dimensions transversales de ce tronçon mesurées selon deux directions sensiblement perpendiculaires, ces moyens de détection s'étendant transversalement à ce tronçon en regard de toute sa largeur interne et de part et d'autre de celle-ci, cette méthode étant également **caractérisée en ce que** l'on choisit pour la paroi dudit tronçon de comptage (4a) un matériau qui est sensiblement transparent à la longueur d'onde des particules émises par ledit fluide.

2. Méthode de comptage de particules selon la revendication 1, **caractérisée en ce que** ces particules sont des photons émis par fluorescence.

3. Méthode de comptage de particules selon la revendication 1, **caractérisée en ce que** ces particules sont des électrons ou des positons émis par radioactivité bêta, ladite paroi présentant une épaisseur e, exprimée en µm, et une masse volumique d, exprimée en g/cm³, telle que le produit e.d soit inférieur à 100 et de préférence inférieur à 50, de sorte minimiser l'atténuation de ces particules en vue de leur comptage.

4. Méthode de comptage de particules selon la revendication 3, **caractérisée en ce que** ledit tronçon de comptage (4a) est à base d'un polymère thermoformé de masse volumique inférieure ou égale à 1,5 g/cm³, de préférence un polyimide de dénomination « Kapton », et **en ce que** ce tronçon présente une paroi d'épaisseur inférieure à 50 µm et de préférence inférieure à 30 µm.

5. Méthode de comptage de particules selon la revendication 3 ou 4, **caractérisée en ce que** ledit fluide est constitué de microéchantillons sanguins qui circulent dans ledit conduit et dans le plasma desquels est dilué un radiotraceur bêta à vie courte, tel que le carbone 11.

6. Méthode de comptage de particules selon la revendication 5, **caractérisée en ce que** lesdits microéchantillons présentent chacun un volume inférieur à 100 µL et de préférence inférieur ou égal à 30 µL, la totalité du volume de chaque microéchantillon étant sensiblement localisée dans ledit tronçon de comptage (4a) en regard desdits moyens de détection (6a), pour que ces microéchantillons se succèdent dans ce tronçon de manière discrétisée dans l'espace et dans le temps.
